# EUROPEAN PATENT SPECIFICATION

(11) **EP 1 373 274 B1**
(45) Date of publication and mention of the grant of the patent: **10.08.2005**
(21) Application number: 01932048.0
(22) Date of filing: 30.03.2001
(51) Int. Cl.: C07D 487/22

(54) **A PROCESS FOR SYNTHESIS OF A PORPHYRIN COMPOUND USING A MOLECULAR SIEVE CATALYST UNDER MICROWAVE IRRADIATION**
VERFAHREN ZUR SYNTHESE EINER PORPHYRINVERBINDUNG MITTELS EINES MOLEKULARSIEBKATALYSATORS UNTER VERWENDUNG VON MIKROWELLENBESTRAHLUNG
PROCEDE DE SYNTHESE D'UN COMPOSE DE PORPHYRINE METTANT EN OEUVRE UN CATALYSEUR A TAMIS MOLECULAIRE SOUS IRRADIATION PAR MICRO-ONDES

(43) Date of publication of application: 02.01.2004
(73) Proprietor: Council of Scientific and Industrial Research, New Delhi 110 001 (IN)
(72) Inventor: KULKARNI, Shivanand Janardan, Indian Inst.Chem.Tec, Andhra Pradesh (IN); RAGHAVAN, Kondapuram Vijaya, Indian Inst.Chem.Tech, Andhra Pradesh (IN); MOTKURI, Radha Kishan, Indian Inst.Chem.Tech., Andhra Pradesh (IN); NAGABANDI, Srinivas, Indian Inst.Chem.Technologies, Andhra Pradesh (IN)
(74) Representative: Jennings, Nigel Robin
(86) International application number: PCT/IN2001/000076
(87) International publication number: WO 2002/079205

(56) References cited:
- DATABASE CA [Online] CHEMICAL ABSTRACTS SERVICE, COLUMBUS, OHIO, US; ALGARRA, FELIPE ET AL: "Condensation of pyrrole with aldehydes in the presence of Y zeolites and mesoporous MCM-41 aluminosilicate: on the encapsulation of porphyrin precursors" retrieved from STN Database accession no. 129:67626 XP002184581 & NEW J. CHEM. (1998), 22(4), 333-338 ,
- DATABASE CA [Online] CHEMICAL ABSTRACTS SERVICE, COLUMBUS, OHIO, US; PETIT, A. ET AL: "Microwave irradiation in dry media: a new and easy method for synthesis of tetrapyrrolic compounds" retrieved from STN Database accession no. 116:214210 XP002184582 cited in the application & SYNTH. COMMUN. (1992), 22(8), 1137-42 ,

## Description

### Field of invention

The present invention relates to a process for synthesis of a porphyrin compound using a molecular sieve catalyst under microwave radiation. More particularly, the present invention relates to a process for synthesis of tetraaryl porphyrin by reacting pyrrole and aromatic aldehyde under microwave irradiation, which is a solvent free system using a specific zeolite catalyst. The invention also relates to a process for the synthesis of tetraphenyl porphyrin by reacting pyrrole with benzaldehyde in presence of zeolite molecular sieves under microwave irradiation. The present invention relates to synthesis of porphyrin compounds over solid acid catalyst.

This invention provides a non-corrosive, eco-friendly process, where the catalyst can be recyclable and reuse for many times, no work up procedure, no-wastage of the compounds (i.e. high atom selectivity), simple sample extraction and high selectivity of products.

### Background of the invention

Porphyrin compounds as well as methods for synthesising the same are well recognised in the art. However, porphyrin compounds and other pyrrole compounds are expensive. For example porphyrine is offered at costs as high as $ 15,000/g. Even though many catalysts such as organic and inorganic acid catalysts are known for the synthesis of porphyrins, the catalysts have at best limited facility for reuse and the yields are very low. Another disadvantage of the prior art processes for the synthesis of porphyrins using such catalysts is that impure corroles are formed making it difficult to separate the pure compound.

The first such report of synthesis of porphyrin molecules under microwave irradiation by A. Petit et al (Synthetic Communication 22(8) (1992) 1139) employed silica alumina, clay and montmorillonite as a catalyst. However, the results were very poor and not more than 10%.

It is therefore important to develop a process for the synthesis of porphyrins with good yield and where catalyst is reusable thereby resulting in economy of costs.

Zeolite catalysts are known in the art for several processes. Zeolites of ZSM series are available from Conteka, Swedan. Methods for producing them are described in detail in U.S. Pat. No. 3,702,886 (ZSM-5). C.T. Kresge, M.E. Leonowicz, W.J. Roth, JC. Vartuti and J.S. Beck, Nature 359 (1992) describe the synthesis of MCM-41 by an aqueous solution of aluminum isopropoxide An aqueous solution of sodium hydroxide (0.3g) was added to aluminum isopropoxide (0.38 g) in 50 ml beaker and stirred in hot conditions, till a clear solution was formed Then 9.4 ml of tetraethyl ammonium hydroxide (TEAOH) and Ludox colloidal silica (9.26 g) were added drop wise white stirring at room temperature. Then hexadecyl tri-methylammonium bromide (10.55 g) was added slowly to the above solution. The pH of the mixture was maintained at 11.0-11.5. finally, the gel mixture was transferred into an autoclave and heated at 100°C for 24 h. The solid product was recovered by filtration, washed with deionized water and dried in air. All the as-synthesized samptes were calcined at 773K in air.

### Objects of the invention

The main object of the present invention is to provide a selective, solvent free, eco-friendly, economical process for synthesis of tetraaryl porphyrines.

This and other Objects of the invention have been achieved by using a zeolite moledular sieve as the catalyst for the microwave radiation method for the synthesis of porphyrins.

F Algarra *et al,* New J. Chem. 1998, p333-338 discloses the use of a zeolite structure to encapsulate porphyrins.

### Summary of the invention

Accordingly the present invention provides a process for the synthesis of a tetraaryl porphyrin of the formula 1 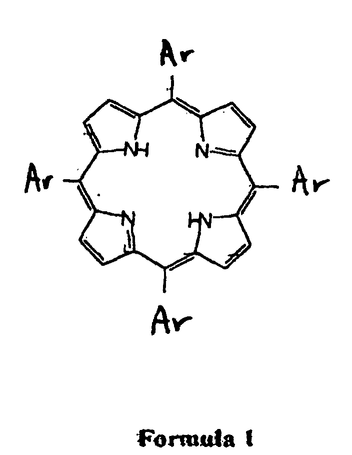 said process comprising reacting the corresponding pyrrole and an aromatic aldehyde ArCHO in a solvent free system under microwave radiation in the presence of a zeolite molecular sieve catalyst to obtain the compound of fomula 1, wherein the aromatic aldehyde is benzaldehyde or of the general formula RPhCHO wherein R in the ortho, meta and para positions is selected from the group consisting of methoxy, NN, dimethylamino, hydroxy and nitro.

In one embodiment of the invention, the zeolite molecular sieve catalyst used is in alkali ion form, ammonium ion form or proton form.

In a further embodiment of the invention, the alkali ion is selected from sodium and postassium.

In another embodiment of the invention, the zeolite molecular sieve catalyst is selected from the group consisting of MCM-41, Al-MCM-41, HY, SAPO-5, ZSM 5 and HZSM-5 (30).

The aromatic aldehyde is of the general formula RPhCHO wherein R in the ortho, meta and para positions is selected from the group consisting of methoxy, N, N, dimethyl, hydroxy and nitro.

In a further embodiment of the invention, the aromatic aldehyde used is selected from the group consisting of benzaldehyde, o/m/p-methoxy benzaldehyde, o/m/p-methyl benzaldehyde, o/m/p-nitro benzaldehyde, m/p-hydroxy benzaldehyde, N,N, dimethyl benzaldehyde, 3,4,5 tri methoxy benzaldehyde.

In a further embodiment of the invention, the pyrrole to aldehyde molar ratio is in the range of 1:1 to 1:4.

In yet another embodiment of the invention, the catalyst is regenerated by burning out the carbon deposited thereon by passing air through the catalyst layer at a temperature in the range of 450°C to 550°C.

In another embodiment of the invention, the yield of the compound of formula 1 is s 23.5 %.

### Detailed description of the invention

Tetraphenyl porphyrines by reacting pyrroles with aromatic aldehyde under microwave irradiation in presence of a catalyst, wherein the catalyst is a commercially available or as synthesized catalyst.

The aromatic aldehydes used in the present invention includes benzaldehyde, o/m/p-methoxy benzaldehyde, o/m/p-methyl benzaldehyde, o/m/p-nitro benzaldehyde, m/p-hydroxy benzaldehyde, N,N, dimethyl benzaldehyde, 3,4,5 tri methoxy benzaldehyde to produce corresponding substituted tetraphenyl porphyrins.

Zeolites used in the present invention are commercially available, and can also be prepared by methods known in the art. The zeolite used in the present invention may be any of an alkali ion form such as sodium, potassium or the like, ammonium ion form and proton form. The alkali ion, however, is not preferably because it lowers the catalytic activity if it remains in the catalyst finally. The microwave power varied from low to high power and the time of heating also varied from 3 minutes to 25 minutes. The catalyst weight can be varied in this reaction from 0.1g to 1g and the pyrrole to aldehyde molar ratio can be varied from 1:1 to 1:4.

In the reaction an equimolar ratio of pyrrole and benzaldehyde were dissolved in a suitable solvent (chloroform/dichloromethane) for thorough mixing and then the sovent was evaporated. To this, the pre-calcined and dried (200°C, 3 h) MCM-41 catalyst was added to and after mixing thouroughly with a glass rod, is subjected to microwave irradiation for 15 minutes with 2 minutes intervels. Then after the reaction, (20 X 5) ml chloroform solvent was added to extract the organic compound and then the catalyst is subjected soxhlet extraction with chloroform. Then porphyrin was seperated by columun chromatography over neutral alumina with hexane as eluent (DCM :Hexane). The quantification was alos done by HPLC and compared with the isolate yields and are characterised by Mass and UV-VIS. Along with the cyclized product, tetraphenyl porphyrin, minor amounts of linear chain condensed products of pyrrole and aldehyde were compounds are also formed.

In place of MCM-41 catalyst when HY was used, a small amount of porphyrin is formed, whereas HZSM-5 (30) yields major amount of porphyrin which may be due to surface reaction. The reaction was compared with SAPO-5 and also with silica alumina catalyst and low conversions and selectivities of the porphyrin molecules under microwave irradiation were observed.

The regeneration of the catalyst is easily effected according to any conventional method. For example the carbon deposited on the catalyst can be burned out by passing air through the catalyst layer at a temperature of 450°C to 550°C.

By using the catalyst of the present invention, as shown, for example, in Example 1, the yield of tetraphenyl porphyrin 23.5%. The yields being shown are as the value calculated based on the conversion of pyrrole. The present invention is described below in more detail referring to Examples, to which the present invention is not limited. By replacing the benzaldehyde with substituted benzaldehydes corresponding tetra substituted porphyrins were formed, which were characterised by UV-VIS spectroscopy.

### EXAMPLE: 1

Mesoporous molecular sieve MCM-41 was synthesized according to C.T. Kresge et al, Nature 359 (1992) 710, as follows.

Solution A was prepared by mixing 0.38g of NaOH, 20ml of water, 0.76 g of Aluminium isoproxide and heated till a clear solution was obtained. After this 9.8 ml of Tetra ethyl ammonium hydroxide was added while cooling the mixture.

Solution B was prepared by mixing 11.6 ml (9.6 g) of 50wt% ludox silica in 50 ml of distilled water the mixture was kept under vigorous stirring until a clear solution formed.

Solution A was added to Solution B under vigorous stirring and kept for stirring for one hour, after that 10.55 g of Hexadecyl trimethyl ammonium bromide (HDTMABr). The pH was adjusted to 10.5.

A stainless steel autoclave having 0.6 liters of volume was charged with the above solution. The autoclave was sealed and heated to 100°C. Hydrothermal synthesis was effected under this condition while continuing stirring for 20 hours. In this period, the inner pressure of the autoclave was 5 to 6 kg/cm².

After completion of the reaction, the reaction mixture was cooled to room temperature and the product was separated by filtration. After repetition of washing and filtration until the concentration of Br⁻ ion in the filtrate became 1 ppm or below, the product was dried at 110°C for 16 hours and then calcined in air at 500°C for 12 hours to elute the surfactant and obtain white crystals of Na form AI-MCM-41. As a result of the measurement of X-ray diffraction, the crystals had a diffraction pattern coincident with that of MCM-41 reported in Nature 1992 by Breck et al.

### EXAMPLE: 2

In a test tube, 1 ml of pyrrole and corresponding volume of benzaldehyde (1: 1 Molar) are dissolved in chloroform solvent and then the solvent was evaporated. To this, pre-calcined and dried 0.5g MCM-41 catalyst was added, and after thorough mixing with a glass rod, subjected to microwave irradiation at a power of 2450 MHz for 15 minutes with 2 minutes intervals. After the reaction, the catalyst was thoroughly washed (20 X 5 ml) with solvent and subjected to soxhlet extraction with chloroform. Porphyrin was separated by column chromatography using neutral alumina and hexane as eluent. The quantification was also done by HPLC and confirmed by isolated yields. Further the products were further confirmed by Uv-Visibte spectroscopy and Mass spectroscopy. Average yields of the products found 23.5% of tetraphenyl porphyrin when pyrrole reacted with benzaldehyde over Al-MCM-41.

### EXAMPLE: 3

The reaction carried out in same manner as in Example 2 with HY catalyst. The yield of tetraphenyl porphyrin is 5%.

### EXAMPLE: 4

The reaction carried out in same manner as in Example 2 with HZSM-5 (30) catalyst. The yield is 28.0%.

### EXAMPLE: 5

The reaction was carried in same manner as in Example 2 except ortho hydroxy benzaldehyde was used instead of benzaldehyde with good yield.

### EXAMPLE: 6

The reaction was carried in same manner as in Example 2 except para methoxy benzaldehyde was used instead of benzaldehyde with good yield.

### EXAMPLE: 7

The reaction was carried in same manner as in Example 2 except N,N dimethyl benzaldehyde was used instead of benzaldehyde with good yield.

### EXAMPLE: 8

The reaction was carried in same manner as in Example 2, except para methyl benzaldehyde was used instead of benzaldehyde with good yield.

### EXAMPLE: 8

The reaction was carried in same manner as in Example 2, except 3,4,5 trimethoxyl benzaldehyde was used instead of benzaldehyde with good yield.

### Advantages of the invention

The present invention provides an improved process that comprises environmentally clean technology with low wastage, easy separable and reusability of the catalyst.
- The catalysts used in this process are easily separable by the simple filtration.
- This process provides an eco-friendly method with higher selectivity.
- A method provides a selective heterogeneous catalyst with longer life.

## Claims

1. A process for the synthesis of a tetraaryl porphyrin of the formula 1 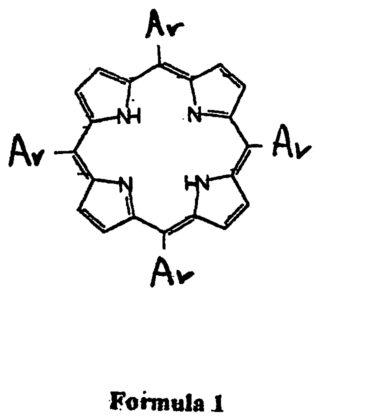 said process comprising reacting the corresponding pyrrole and an aromatic aldehyde ArCHO in a solvent free system under microwave radiation in the presence of a zeolite molecular sieve catalyst to obtain the compound of formula 1, wherein the aromatic aldehyde is benzaldehyde or the general formula RPhCHO wherein R in the ortho, meta and para positions is selected from the group consisting of methoxy, N,N, dimethylamino, hydroxy and nitro.

2. A process as claimed in claim 1 wherein the zeolite molecular sieve catalyst used is in alkali ion form, ammonium ion form or proton form.

3. A process as claimed in claim 2 wherein the alkali ion is selected from sodium and potassium.

4. A process as claimed in claim 1 wherein zeolite molecular sieve catalyst is selected from the group consisting of MCM-41, AL-MCM-41, HY, SAPO-5, ZSM 5 and HZSM-5(30).

5. A process as claimed in any one of claims 1 to 4 wherein the aromatic aldehyde used is selected from the group consisting of benzaldehyde, o/m/p-methoxy benzaldehyde, o/m/p-methyl benzaldehyde, o/m/p-nitro benzaldehyde, m/p-hydroxy benzaldehyde, N,N, dimethyl benzaldehyde and 3,4,5 tri methoxy benzaldehyde.

6. A process as claimed in claim 1 wherein the pyrrole to aldehyde molar ratio is in the range of 1:1 to 1:4.

7. A process as claimed in claim 1 wherein the catalyst is regenerated by burning out the carbon deposited thereon by passing air through the catalyst layer at a temperature in the range of 450°C to 550°C.

8. A process as claimed in claim 1 wherein the time period of heating is in the range of 3 minutes to 30 minutes.

9. A process as claimed in claim 1 wherein the yield of the compound of formula 1 is 23.5%.

## Patentansprüche

1. Verfahren für die Synthese eines Tetraaryl-Porphyrins der Formel 1 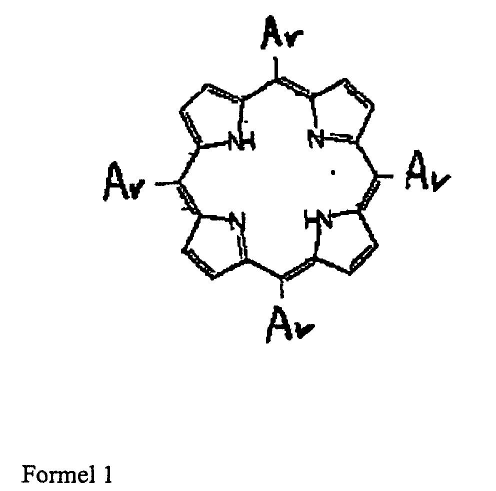 wobei das vorerwähnte Verfahren das Umsetzen des entsprechenden Pyrrols und eines aromatischen Aldehyds ArCHO in einem lösungsmittelfreien System unter Mikrowellenbestrahlung in Gegenwart eines Zeolit-Molekularsiebkatalysators zur Bildung der Verbindung von Formel 1 aufweist, wobei das aromatische Aldehyd Benzaldehyd von der allgemeinen Formel RPhCHO ist, worin R in der Ortho-, Meta- und Parastellung aus der Gruppe gewählt wird, die aus Methoxy, N,N-Dimethylamino, Hydroxy und Nitro besteht.

2. Verfahren nach Anspruch 1, **dadurch gekennzeichnet, dass** der verwendete Zeolit-Molekularsiebkatalysator in der Alkali-Ionenform, Ammonium-Ionenform oder Protonenform ist.

3. Verfahren nach Anspruch 2, **dadurch gekennzeichnet, dass** das Alkaliion aus Natrium und Kalium ausgewählt wird.

4. Verfahren nach Anspruch 1, **dadurch gekennzeichnet, dass** der Zeolit-Molekularsiebkatalysator aus der Gruppe gewählt wird, die aus MCM-41, AL-MCM-41, HY, SAPO-5, ZSM 5 und HZSM-5(30) besteht.

5. Verfahren nach einem der Ansprüche 1 bis 4, **dadurch gekennzeichnet, dass** der aromatische Aldehyd aus der Gruppe gewählt ist, die aus Benzaldehyd, o/m/p-Methoxybenzaldehyd, o/m/p-Methylbenzaldehyd, o/m/p-Nitrobenzaldehyd, m/p-Hydroxybenzaldehyd, N,N-Dimethylbenzaldehyd und 3,4,5-Trimethoxybenzaldehyd besteht.

6. Verfahren nach Anspruch 1, **dadurch gekennzeichnet, dass** das molare Verhältnis von Pyrrol zu Aldehyd im Bereich von 1:1 bis 1:4 liegt.

7. Verfahren nach Anspruch 1, **dadurch gekennzeichnet, dass** der Katalysator durch Ausbrennen des Kohlenstoffs, der sich darauf abgesetzt hat, bei Durchgang von Luft durch die Katalysatorschicht bei einer Temperatur im Bereich von 450 °C bis 550 °C regeneriert wird.

8. Verfahren nach Anspruch 1, **dadurch gekennzeichnet, dass** die Dauer der Erhitzung im Bereich von 3 Minuten bis 30 Minuten liegt.

9. Verfahren nach Anspruch 1, **dadurch gekennzeichnet, dass** die Ausbeute der Verbindung von Formel 1 23,5 % beträgt.

## Revendications

1. Procédé de synthèse d'une tétraarylporphyrine de formule 1 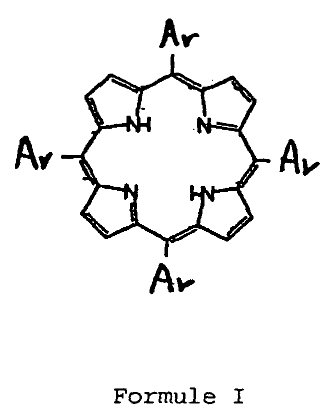 ledit procédé comprenant la mise en réaction du pyrrole correspondant et d'un aldéhyde aromatique ArCHO dans un système sans solvant sous irradiation par micro-ondes en présence d'un catalyseur à tamis moléculaire zéolitique pour obtenir le composé de formule 1, **caractérisé en ce que** l'aldéhyde aromatique est un benzaldéhyde de formule générale RPhCHO dans laquelle R en positions ortho, méta et para est sélectionné parmi le groupe constitué de méthoxy, N,N-diméthylamino, hydroxy et nitro.

2. Procédé selon la revendication 1, **caractérisé en ce que** le catalyseur à tamis moléculaire zéolitique utilisé est sous forme d'un ion alcalin, de l'ion d'ammonium ou de proton.

3. Procédé selon la revendication 2, **caractérisé en ce que** l'ion alcalin est sélectionné parmi le sodium et le potassium.

4. Procédé selon la revendication 1, **caractérisé en ce que** le catalyseur à tamis moléculaire zéolitique est sélectionné parmi le groupe constitué de MCM-41, AL-MCM-41, HY, SAPO-5, ZSM 5 et HZSM-5(30).

5. Procédé selon l'une quelconque des revendications 1 à 4, **caractérisé en ce que** l'aldéhyde aromatique utilisé est sélectionné parmi le groupe constitué de benzaldéhyde, o/m/p-méthoxybenzaldéhyde, o/m/p-méthylbenzaldéhyde, o/m/p-nitrobenzaldéhyde, m/p-hydroxybenzaldéhyde, N,N-diméthylbenzaldéhyde et 3,4,5-triméthoxybenzaldéhyde.

6. Procédé selon la revendication 1, **caractérisé en ce que** le rapport molaire du pyrrole à l'aldéhyde est dans la plage de 1:1 à 1:4.

7. Procédé selon la revendication 6, **caractérisé en ce que** le catalyseur est régénéré par combustion du carbone déposé dessus en passant de l'air à travers la couche du catalyseur à une température dans la plage de 450°C à 550°C.

8. Procédé selon la revendication 1, **caractérisé en ce que** la période de temps de chauffage est dans la plage de 3 minutes à 30 minutes.

9. Procédé selon la revendication 1, **caractérisé en ce que** le rendement en composé de formule 1 est de 23,5%.
